# EUROPEAN PATENT APPLICATION

(11) **EP 4 306 070 A1**
(43) Date of publication of application: **17.01.2024**
(21) Application number: 22187375.5
(22) Date of filing: 28.07.2022
(51) Int. Cl.: A61B 18/26

(54) **DIRECTABLE LASER CATHETER SONIC WAVE WITH FOCUSING CHAMBER**

(30) Priority: 15.07.2022 US 202263389378 P
(71) Applicant: Koninklijke Philips N.V., 5656 AG Eindhoven (NL)
(72) Inventor: BRUBAKER, Clint, Eindhoven (NL)
(74) Representative: Philips Intellectual Property & Standards

(57) **Abstract**

An intravascular therapy device (10) includes a laser catheter (12) including at least one optical fiber (14) extending through the laser catheter which terminates at a laser output aperture (16); a containment sheath (18) surrounding the laser catheter and configured to contain a contrast mixture (20), the containment sheath being longitudinally movable respective to the laser catheter to cover or uncover the laser aperture, whereby laser light output at the laser output aperture being effective to induce sonic energy in the contrast mixture when the containment sheath covers the laser aperture; and a focusing chamber (22) arranged over the containment sheath, the focusing chamber including at least one sonic energy aperture (24) at a side of the focusing chamber, the focusing chamber configured to direct the sonic energy out the at least one sonic energy aperture.

## Description

### TECHNICAL FIELD

The following relates generally to the catheter arts, vascular therapy, lesion treatment arts, and related arts.

### BACKGROUND

In catheter-based vascular therapy, a laser catheter utilizes 308 nm laser light transmitted down a catheter to the distal tip where the light can ablate tissue. The laser light can be used with a contrast mixture. The laser light breaks down the contrast and, in the process, generates a sonic wave. This sonic wave emanates in a circular wave around the tip of the laser catheter. When emitted in a calcified lesion, the sonic wave can create fractures in the calcium. However, because of the circular nature of the wave, if there is a specific section that does not have a calcified lesion that fully encapsulates the vessel, then a large portion of the generated energy from the pressure wave may bypass the targeted calcium.

Current laser catheter devices can generate a pressure wave in a circular wave around the tip of the laser catheter. However, because of the circular nature of the wave, if there is a specific section that does not have a calcified lesion that fully encapsulates the vessel, then a large portion of the generated energy from the pressure wave may bypass the target calcium. Using a device that can focus this pressure wave to the desired section may potentially increase the clinical effectiveness of the device and allow for more treatment options. However, the device would also amplify the amplitude of the pressure wave in a specific direction enabling the device to fracture a greater amount of the calcium deposits potentially.

The following discloses certain improvements to overcome these problems and others.

### SUMMARY

In some embodiments disclosed herein, an intravascular therapy device includes a laser catheter including at least one optical fiber extending through the laser catheter which terminates at a laser output aperture; a containment sheath surrounding the laser catheter and configured to contain a contrast mixture, the containment sheath being longitudinally movable respective to the laser catheter to cover or uncover the laser aperture, whereby laser light output at the laser output aperture being effective to induce sonic energy in the contrast mixture when the containment sheath covers the laser aperture; and a focusing chamber arranged over the containment sheath, the focusing chamber including at least one sonic energy aperture at a side of the focusing chamber, the focusing chamber configured to direct the sonic energy out the at least one sonic energy aperture.

In some embodiments the focusing chamber may be configured to move longitudinally along a length of the laser catheter to longitudinally align the at least one sonic energy aperture with the laser output aperture.

In some embodiments disclosed herein, an intravascular therapy method includes inserting a catheter assembly comprising a laser catheter surrounded by a containment sheath and further comprising a focusing chamber into a blood vessel to position the focusing chamber at an intravascular treatment site having a calcified occlusion; and after the inserting, treating the calcified occlusion by concurrently: inputting laser light into the laser catheter to emit the laser light at a laser aperture located inside the focusing chamber; flowing a contrast mixture into the focusing chamber via the containment sheath whereby the laser light induces sonic energy in the contrast mixture in the containment sheath; and by the focusing chamber, directing the sonic energy out a sonic energy aperture of the focusing chamber.

One advantage resides in directing sonic energy to an occlusion in a blood vessel.

Another advantage resides in using a focusing chamber with a laser catheter to direct sonic energy to an occlusion in a blood vessel.

Another advantage resides in using a focusing chamber with an aperture to direct sonic energy to an occlusion in a blood vessel.

According to some embodiments an advantage resides in moving a focusing chamber along a length of a laser catheter to direct sonic energy to an occlusion in a blood vessel. In some embodiments the focusing chamber may be configured to move longitudinally along a length of the laser catheter to longitudinally align the at least one sonic energy aperture with the laser output aperture.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically illustrates a vascular therapy device in accordance with the present disclosure.
FIGURES 2-4 show embodiments of a focusing chamber of the device of FIGURE 1.
FIGURE 5 diagrammatically illustrates a vascular therapy method using the device of FIGURE 1.

### DETAILED DESCRIPTION

The following relates to an intravascular laser ablation system in which a contrast mixture is broken down by laser light, creating a sonic wave that debulks hardened calcium deposits. The contrast mixture also serves to provide imaging contrast. To produce the sonic wave, the contrast agent includes a substance that absorbs the laser light (for example, iodine in the case of 308 nm excimer laser light), and an outer sheath surrounding the laser catheter serves to contain the contrast mixture. The outer sheath may be retracted to expose the laser light directly to the clot to operate in conventional laser ablation mode, or moved forward to contain the contrast agent in sonic wave-mediated calcium debulking. The outer sheath is typically a plastic material that provide confinement of the contrast mixture but transmits the sonic wave.

The following provides a sonic energy focusing chamber in the form of a stainless steel hypotube (i.e. a tube with thin walls) with an opening in the side. The sonic energy focusing chamber acts to concentrate the sonic wave and preferentially emit it out of the side opening. This both increases the sonic energy directed out the opening and reduces or eliminates the sonic energy flowing in other directions. By rotating the catheter bearing the sonic energy focusing chamber, a directional emission of the sonic energy is achievable.

In one embodiment, the sonic energy focusing chamber is mounted at the end of the existing outer sheath used to contain the contrast mixture during calcium debulking. In another embodiment, the sonic energy focusing chamber is mounted on a third concentric catheter that surrounds the outer sheath (which in turn surrounds the laser catheter).

A further modification entails providing for rotation of the catheter bearing the sonic energy focusing chamber. This is straightforward to implement by adding a knob or the like to enable rotation of the outer sheath or of the additional sheath added around the outer sheath.

The illustrative sonic energy focusing chamber is a tube, but other geometries for the sonic energy focusing chamber are contemplated, such as shaping the sonic energy focusing chamber to enhance the concentration of the sonic energy. The illustrative sonic energy focusing chamber has an open distal end, but in a variant embodiment this could be closed off by a normally-closed hinged door or a translatable door that would be open during passage of the sonic energy focusing chamber over the laser catheter but would spring shut when the sonic energy focusing chamber is moved past the end of the laser catheter.

Further variants include the sonic wave being generated by another mechanism, such as rapid reciprocation of the laser catheter within an outer sheath.

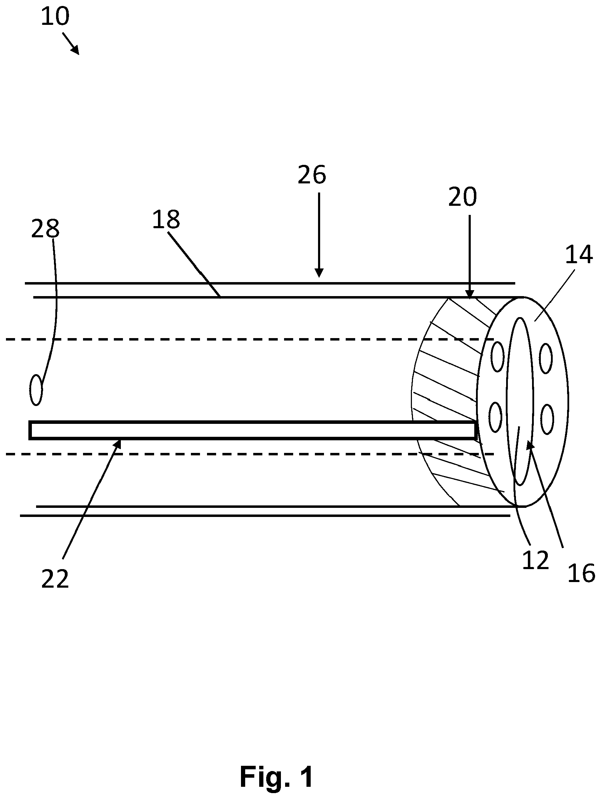

With reference to FIGURE 1, an illustrative intravascular therapy device 10 insertable into a blood vessel for treating a lesion (or a clot, or an occlusion, and so forth) in the blood vessel is diagrammatically shown. As shown in FIGURE 1, the device 10 includes a laser catheter 12 including at least one optical fiber 14 (shown in FIGURE 1 as a ring of optical fibers) extending through the laser catheter 12 which terminates at a laser output aperture 16.

A containment sheath 18 surrounds the laser catheter 12, and is configured to contain a contrast mixture 20 (i.e., iodine) (schematically shown in FIGURE 1 with cross-hatching). The containment sheath 18 is longitudinally movable respective to the laser catheter 12 to cover or uncover the laser aperture 16. Laser light that is output at the laser output aperture 16 is effective to induce sonic energy in the contrast mixture 20 when the containment sheath 18 covers the laser aperture 16. The laser light can be generated by a laser generator (not shown), and can have a wavelength of 308 nm.

FIGURE 1 also shows a control element 28 (i.e., a knob) disposed at a proximal end of the device 10. The control element 28 is configured to rotate the focusing chamber 22 around the laser catheter 18.

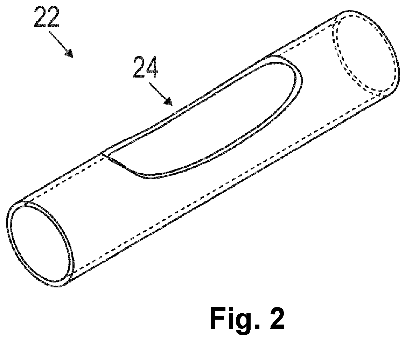

With containing reference to FIGURE 1, FIGURE 2 shows a focusing chamber 22 arranged over the containment sheath 18 (the focusing chamber 22 is schematically shown in FIGURE 1 as an elongated rectangle). The focusing chamber 22 comprises a stainless steel tube. The focusing chamber 22 includes at least one sonic energy aperture 24 at a side of the focusing chamber 22. The focusing chamber 22 is configured to direct the sonic energy out the at least one sonic energy aperture 24. This focusing chamber 22 would not be encompassing the circumference of the laser catheter 12. The portion of the laser catheter 12 that does not have the focusing chamber 22 would allow the pressure wave to exit more easily. In one example, the focusing chamber 22 is attached to an outside of the containment sheath 18. In another example, the focusing chamber 22 is attached to an additional sheath 26 (FIGURE 1) surrounding the containment sheath 18.

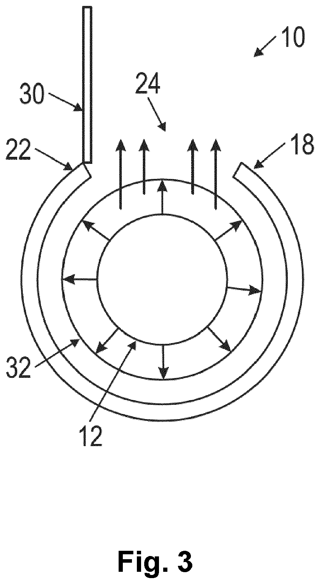

The at least one sonic energy aperture 24 comprises (i) at least one opening, or (ii) at least one sonic energy-transmissive window. In some embodiments, as shown in FIGURE 3, the focusing chamber 22 includes a normally-closed hinged door 30 (the door 30 shown in FIGURE 3 is open) at a distal portion of the focusing chamber 22, and configured to cover the at least one sonic energy aperture 24 when the sonic energy is not transmitted through the laser catheter 12. To direct the sonic energy out of the at least one sonic energy aperture 24, the focusing chamber 22 is configured to move longitudinally along a length of the laser catheter 12 to longitudinally align the at least one sonic energy aperture 24 with the laser output aperture 16 of the laser catheter 12. In alternative embodiments, the normally-closed hinged door 30 in FIGURE 3 can be substituted at a distal portion of the focusing chamber 22 by a normally-closed door that is operable by longitudinal translation or lateral displacement of the door either inside or outside adjacent to the wall of the focusing chamber 22, and configured to cover the at least one sonic energy aperture 24 when the sonic energy is not transmitted through the laser catheter 12. In some of the embodiments the shape of the door is such that it is complementary to the shape of the focusing chamber in the normally-closed position, such that the cross section of the focusing chamber proximal or distal to the sonic energy aperture is substantially similar to that of the cross section at the location of the sonic energy aperture when it is covered by the door.

As shown in FIGURE 3, the focusing chamber 22 allows the device 10 to potentially have more effective treatment in areas with heavy calcium and reduce potential treatment in areas that treatment is not wanted. The focusing chamber 22 can allow a pressure wave (designated as reference character 32) generated within the focusing chamber 22 to be directed to the desired treatment area by a physician. Also, the pressure wave can be amplified in the desired direction and reduced in areas that may not want to be treated as much.

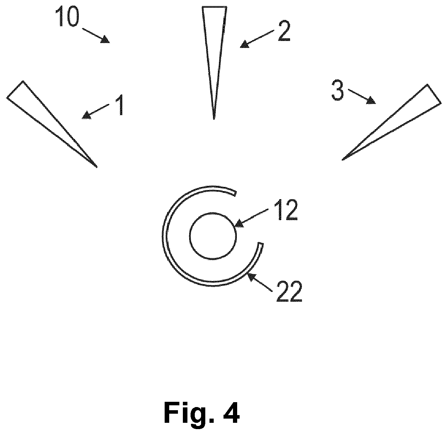

FIGURE 4 shows a hydrophone testing schematic of the device 10. As shown in FIGURE 4, three hydrophones (designated with reference characters 1, 2, 3) were positioned around the catheter at 45 degrees offset from the center of the laser catheter 12. Hydrophone 3 was directly inline (i.e., 0 degrees) with the exit of the focusing chamber 22, hydrophone 2 was 45 degrees offset from the exit of the focusing chamber 22, and hydrophone 1 was 90 degrees offset from the exit. The test was run without the focusing chamber 22 around the tip of the laser catheter 12 as a control and with the focusing chamber 22 around the tip. Results from the testing showed that the amplitude of the hydrophones changed by -51% for hydrophone 1, +20% for hydrophone 2, and +55% for Hydrophone 3. These results support that using the focusing chamber 22 amplifies the magnitude of the sonic wave in the direction it is pointing and dampens the wave in areas away from the exit.

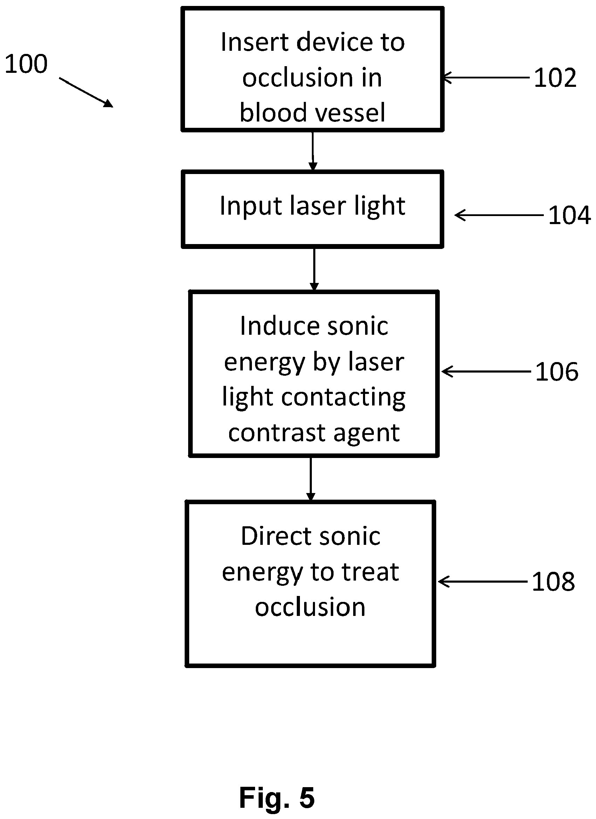

Referring to FIGURE 5, an illustrative embodiment of an intravascular therapy method or process 100 using the device 10 is diagrammatically shown as a flowchart. At an operation 102, the device 10 is inserted into a blood vessel to position the focusing chamber 22 at an intravascular treatment site having a calcified occlusion. The calcified occlusion is then treated. To do so, at an operation 104, laser light is input into the laser catheter 12 to emit the laser light at the laser aperture 24 located inside the focusing chamber 22. At an operation 106, the contrast mixture 20 is flowed from the containment sheath 18 into the focusing chamber 22. The laser light induces sonic energy in the contrast mixture 20 in the containment sheath 18. At an operation 108, the focusing chamber 22 directs the sonic energy out of the sonic energy aperture 24 and to the calcified occlusion.

The device 10 could potentially be used in the peripheral, coronary, or any part of the body in the application of fracturing calcium or hard material in the body. The device 10 can potentially be used for any treatment that requires breaking up a hard material via pressure wave.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. An intravascular therapy device (10), comprising:
a laser catheter (12) including at least one optical fiber (14) extending through the laser catheter which terminates at a laser output aperture (16);
a containment sheath (18) surrounding the laser catheter and configured to contain a contrast mixture (20), the containment sheath being longitudinally movable respective to the laser catheter to cover or uncover the laser aperture, whereby laser light output at the laser output aperture being effective to induce sonic energy in the contrast mixture when the containment sheath covers the laser aperture; and
a focusing chamber (22) arranged over the containment sheath, the focusing chamber including at least one sonic energy aperture (24) at a side of the focusing chamber, the focusing chamber configured to direct the sonic energy out the at least one sonic energy aperture.

2. The device (10) of claim 1, wherein the at least one sonic energy aperture (24) of the focusing chamber (22) includes at least one opening or at least one sonic energy-transmissive window.

3. The device (10) of either one of claims 1 and 2, wherein the focusing chamber (22) comprises a stainless steel tube.

4. The device (10) of any one of claims 1-3, wherein the focusing chamber (22) is attached to an outside of the containment sheath (18).

5. The device (10) of any one of claims 1-3, further comprising an additional sheath (26) surrounding the containment sheath (18), the focusing chamber (22) attached to the additional sheath.

6. The device (10) of any one of claims 1-5, further comprising:
a control element (28) disposed at a proximal end of the device and configured to rotate the focusing chamber (22) around the laser catheter (12).

7. The device (10) of any one of claims 1-6, wherein the focusing chamber (22) is configured to move longitudinally along a length of the laser catheter (12) to longitudinally align the at least one sonic energy aperture (24) with the laser output aperture (16).

8. The device (10) of any one of claims 1-7, wherein the focusing chamber (22) includes a normally-closed hinged door (30) at a distal portion of the focusing chamber.

9. The device (10) of any one of claims 1-7, wherein the focusing chamber (22) comprises a normally-closed door at a distal portion of the focusing chamber, operable by longitudinal translation or lateral displacement of the door either inside or outside adjacent to a wall of the focusing chamber (22).

10. The device (10) of claim 8 or 9, wherein the door is shaped such that the cross section of the focusing chamber proximal or distal to the sonic energy aperture is substantially similar to that of the cross section at the location of the sonic energy aperture when it is covered by the door.

11. An intravascular therapy method (100), comprising:
inserting a catheter assembly comprising a laser catheter (12) surrounded by a containment sheath (18) and further comprising a focusing chamber (22) into a blood vessel to position the focusing chamber at an intravascular treatment site having a calcified occlusion; and
after the inserting, treating the calcified occlusion by concurrently:
inputting laser light into the laser catheter to emit the laser light at a laser aperture (24) located inside the focusing chamber;
flowing a contrast mixture (20) into the focusing chamber via the containment sheath whereby the laser light induces sonic energy in the contrast mixture in the containment sheath; and
by the focusing chamber, directing the sonic energy out a sonic energy aperture of the focusing chamber.

12. The method (100) of claim 11, further comprising:
aligning the laser aperture (24) located inside the focusing chamber (22) with a laser output aperture (16) of the laser catheter (12) to direct the sonic energy.

13. The method (100) of either one of claims 11 and 12, further comprising:
rotating the focusing chamber (22) around the laser catheter (12) to direct the sonic energy.

14. The method (100) of any one of claims 11-13, wherein the laser light has a frequency of 308 nm.

15. The method (100) of any one of claims 11-14, wherein the contrast agent (20) comprises iodine.
